# EUROPEAN PATENT SPECIFICATION

(11) **EP 2 535 065 B1**
(45) Date of publication and mention of the grant of the patent: **19.07.2017**
(21) Application number: 11742208.9
(22) Date of filing: 08.02.2011
(51) Int. Cl.: A61M 1/14, A61M 1/34, A61M 1/36

(54) **DIALYZER**
DIALYSEGERÄT
DIALYSEUR

(30) Priority: 09.02.2010 JP 2010026622
(43) Date of publication of application: 19.12.2012
(73) Proprietor: Nikkiso Company Limited, Tokyo 150-6022 (JP)
(72) Inventor: HASEGAWA, Shinya, Makinohara-shi Shizuoka 421-0496 (JP); TANAKA, Kensaku, Makinohara-shi Shizuoka 421-0496 (JP)
(74) Representative: von Hirschhausen, Helge
(86) International application number: PCT/JP2011/052595
(87) International publication number: WO 2011/099464

(56) References cited:
- DE-A1- 4 240 681
- JP-A- 11 267 197
- JP-A- 2001 112 863
- JP-A- 2004 313 522
- US-A- 5 895 368
- US-A1- 2005 131 331

## Description

### Field of the Invention

The present invention relates to a dialysis apparatus to allow a dialyzer (blood purification instrument) connected to a blood circuit to perform the dialysis.

### Description of Background Art

In general, the dialysis apparatus used for the hemodialysis treatment is provided with a dialysate introducing line and a dialysate discharging line respectively for supplying the dialyzer connected to the blood circuit with dialysate and discharging dialysate containing blood waste materials produced by dialysis. The tips of these dialysate introducing line and the dialysate discharging line are connected respectively to a dialysate introducing port and a dialysate discharging port of the dialyzer.

In a blood purification instrument (dialyzer) applied to the hemodialysis filtering (HDF) and using dialysate as infusion solution (hereinafter referred to as "online HDF"), it is necessary to infuse dialysate into blood of a patient by an amount of ultrafiltration. Accordingly, it is proposed, for example in Patent Document 1 below, a dialysis apparatus shown in Fig. 6 comprising a blood circuit having a dialyzer 101, an arterial blood circuit 102 arranged therein a blood pump 104 and a venous blood circuit 103, a dialysate introducing line L1 for introducing the dialysate supplied by a pump 105 to the dialyzer 101, a dialysate discharging line L2 for discharging the dialysate from the dialyzer 101, a bypass line L4 for connecting the dialysate introducing line L1 and the dialysate discharging line L2 not via the dialyzer 101, and a dialysate infusing line L3.

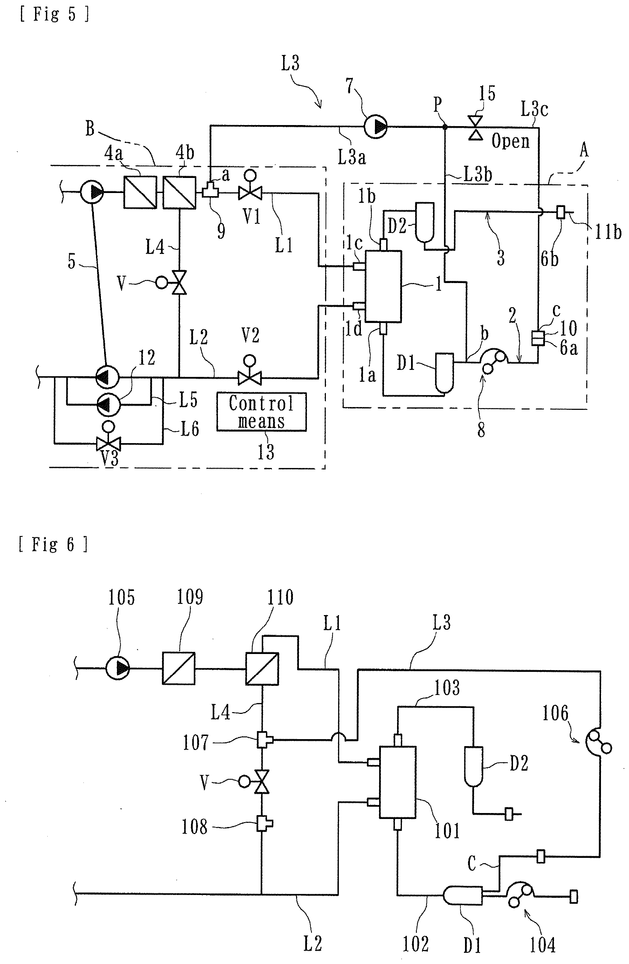

Such a dialysis apparatus of the online HDF is structured so that it ultrafiltrates a predetermined water from blood during the hemodialysis treatment and then replaces the ultrafiltrated water with dialysate with supplying the dialysate from the dialysate infusing line L3 and thus has a dialysate infusing pump 106 for controlling the flow rate through the dialysate infusing line L3 during the dialysate infusion. In Fig. 6, reference numerals 109 and 110 denote filters for filtering dialysate flowing through the dialysate introducing line L1 and a reference character "V" denotes an electromagnetic valve. In addition, air trap chambers D1, D2 are arranged respectively on the arterial blood circuit 102 and the venous blood circuit 103.

The dialysis treatment has been performed by connecting the base end of the dialysate infusing line L3 to one T-tube 107, connecting the tip end of the dialysate infusing line L3 to the tip end of a tube "c" extending from the air trap chamber D1 of the arterial blood circuit 102, and infusing the dialysate from the air trap chamber D1 by an amount of water to be filtered (pre-dialysate infusion). The dialysate infusion (post-dialysate infusion) can be also performed by connecting the tip end of the dialysate infusing line L3 to the tip end of a tube extended from the air trap chamber D2.

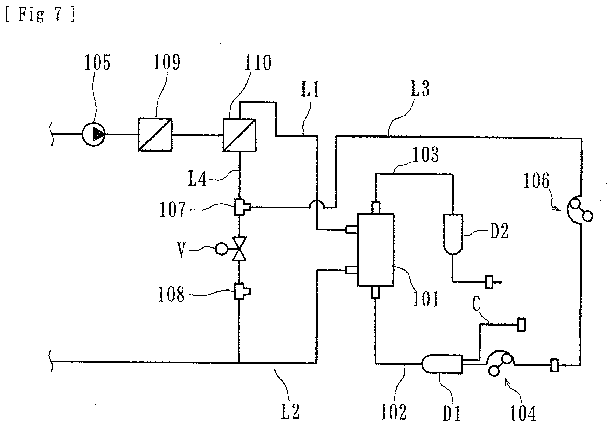

The blood-return after the dialysis treatment can be performed in such a dialysis apparatus as shown in Fig. 7 by connecting the tip end of the dialysate infusing line L3 to the tip end (i.e. similarly to the priming, a connector after removal of an arterial puncture needle) of the arterial blood circuit 102 and supplying the dialysate from the pump 15 with keeping a puncture needle at the tip end of the venous blood circuit 103 punctured into a patient. This enables blood remained in the blood circuits to be replaced with dialysate and the remained blood to be returned to a body of a patient from the venous puncture needle. During this blood-returning process, it is also necessary to drive both the dialysate infusing pump 106 and the blood pump 104 to the normal rotational direction.

### Documents of Prior Art

### Patent Document

Patent Document 1 Japanese Laid-open Patent Publication No. 313522/2004

US 2005/131331 A1.

### Disclosure of the Invention

The invention is defined in the appended claims.

### Problems to be solved by the Invention

However, there are following problems in the dialysis apparatus of the prior art.

In usual, there is an error in the flow rate caused by general purpose pumps such as blood pump and dialysis pump relative to the set flow rate (discharging amount). Accordingly, it is afraid that there would be caused an excessive positive pressure in a flow route between the blood pump 104 and the dialysate infusing pump 106 and the flow route would be broken when, for example, the discharging amount of the blood pump 104 is lower than that of the dialysate infusing pump 106 in the blood-returning process if the blood pump 104 are connected in series as shown in Fig. 7.

It is, therefore, an object of the present invention to provide a dialysis apparatus which can prevent the generation of excessive positive pressure in the flow route between the blood pump and the dialysate infusing pump although there would be caused an error in the flow rate of the blood pump and the dialysate infusing pump during the blood-returning process.

### Means for solving the Problems

For achieving the object of the present invention, there is provided, according to aspect 1, a dialysis apparatus comprising a blood purification instrument containing therein a blood purification membrane and formed with a blood introducing port, a blood discharging port, a dialysate introducing port and a dialysate discharging port for performing the dialysis purification by contacting blood with dialysate through the blood purification membrane; an arterial blood circuit, the base end of which being connected to the blood introducing port of the blood purification instrument and having a blood pump arranged thereon; a venous blood circuit, the base end of which being connected to the blood discharging port; a dialysate introducing line connected to the dialysate introducing port of the blood purification instrument for introducing dialysate to the blood purification instrument; a dialysate discharging line connected to the dialysate discharging port of the blood purification instrument for discharging dialysate from the blood purification instrument; and a dialysate supplying device for supplying prepared dialysate to the dialysate introducing line characterized in that the dialysis apparatus further comprises: a dialysate infusing line, one end of which being connected to the dialysate introducing line or the dialysate discharging line, the other end-side being branched out at a branch point into two flow routes respectively having a first branch end and a second branch end, the first branch end being able to be connected to the arterial blood circuit or the venous blood circuit, and the second branch end being able to be connected to the tip end of the arterial blood circuit during the blood-returning process, and a dialysate infusing pump arranged on the dialysate infusing line at a connection-side of the dialysate infusing line relative to the dialysate introducing line or the dialysate discharging line from the branch point for supplying the dialysate of the dialysate introducing line or the dialysate discharging line to the first branch end and the second branch end. The dialysis apparatus further comprises a control device for synchronously controlling the blood pump and the dialysate infusing pump so that the rotational speed of the dialysate infusing pump is higher than that of the blood pump by a predetermined ratio during the blood-returning process.

Aspect 2 is a dialysis apparatus of aspect 1 wherein a valve device for cutting off or opening the supply of dialysate is arranged on the dialysate infusing line between the branch point and the second branch end.

Aspect 3 is a dialysis apparatus of aspect 1 or 2 wherein the first branch end can be connected to an arterial air-trap chamber arranged on the arterial blood circuit or a venous air-trap chamber arranged on the venous blood circuit.

Aspect 4 is a dialysis apparatus of aspect 1 wherein the predetermined ratio is about 10%.

Aspect 5 is a dialysis apparatus of aspect 1 or 4 wherein the control device controls the blood pump and the dialysate infusing pump so that both the blood pump and the dialysate infusing pump are driven at the start of the blood-return and only the dialysate infusing pump is driven with stopping the blood pump after the lapse of a predetermined time.

### Effects of the Invention

According to aspect 1, since the dialysis apparatus comprises a dialysate infusing line, one end of which being connected to the dialysate introducing line or the dialysate discharging line, the other end-side being branched out at a branch point into two flow routes respectively having a first branch end and a second branch end, the first branch end being able to be connected to the arterial blood circuit or the venous blood circuit, and the second branch end being able to be connected to the tip end of the arterial blood circuit during the blood-returning process, it is possible, during the blood-returning process, to allow the dialysate to be flowed through both the flow route of the dialysate infusing line between the branch point and the second branch end and the flow route between the branch point and the first branch end. Accordingly, the dialysate can flow through the flow route between the branch point and the first branch end of the dialysate infusing line even when the flow rate (discharging amount) of the blood pump is lower than that of the dialysate infusing pump due to the error in flow rate of the blood pump and the dialysate infusing pump during the blood-returning process, and thus the generation of excessive positive pressure in the flow route between the blood pump and the dialysate infusing pump can be prevented.

According to aspect 2, since a valve device for cutting off or opening the supply of dialysate is arranged on the dialysate infusing line between the branch point and the second branch end, it is possible to surely prevent the dialysate from being discharged from the second branched end on the dialysate infusion time during dialysis treatment with the dialysate flow being cut off before the blood-return and opened during the blood-return by the valve device.

According to aspect 3, since the first branch end can be connected to an arterial air-trap chamber arranged on the arterial blood circuit or a venous air-trap chamber arranged on the venous blood circuit, it is possible to perform the bubble removal on feeding the dialysate to the arterial blood circuit or the venous blood circuit during the dialysate infusion into the arterial blood circuit.

According to the invention as described in claim 1, since the dialysis apparatus further comprises a control device configured to synchronously control the blood pump and the dialysate infusing pump so that the rotational speed of the dialysate infusing pump is higher than that of the blood pump by a predetermined ratio during the blood-returning process, it is possible to prevent back flow of the blood through the flow route between the branch point and the first branch end of the dialysate infusing line even though error would be caused in flow rate of the blood pump and the dialysate infusing pump during the blood-returning process.

According to aspect 4, since the predetermined ratio is about 10%, it is possible to sufficiently absorb the error (about 10%) usually caused in general purpose pumps such as blood pumps and dialysate infusing pumps.

According to aspect 5, since the control device controls the blood pump and the dialysate infusing pump so that both the blood pump and the dialysate infusing pump are driven at the start of the blood-return and only the dialysate infusing pump is driven with stopping the blood pump after the lapse of a predetermined time, it is possible to reduce the consumption of dialysate during the blood-return and the time of blood-return.

### Brief Description of the Drawings

[Fig. 1] A schematic view showing the dialysis apparatus of one embodiment of the present invention;
[Fig. 2] A schematic view showing the dialysate infusing line of the dialysis apparatus of Fig. 1;
[Fig. 3] A schematic view showing the connection of dialysate infusing line during the blood-return in the dialysis apparatus of Fig. 1;
[Fig. 4] A schematic view showing the connection of dialysate infusing line during the blood-return in the dialysis apparatus of one modification of Fig. 1;
[Fig. 5] A schematic view showing the connection of dialysate infusing line during the blood-return in the dialysis apparatus of the other modification of Fig. 1;
[Fig. 6] A schematic view showing a dialysis apparatus of the prior art in a condition of the hemodialysis treatment or the dialysate infusion; and
(Fig. 7] A schematic view showing the dialysis apparatus of the prior art of Fig. 6 in a blood-return condition.

### Modes for carrying out the Invention

A preferable embodiment of the present invention will be hereinafter described with reference to the drawings.

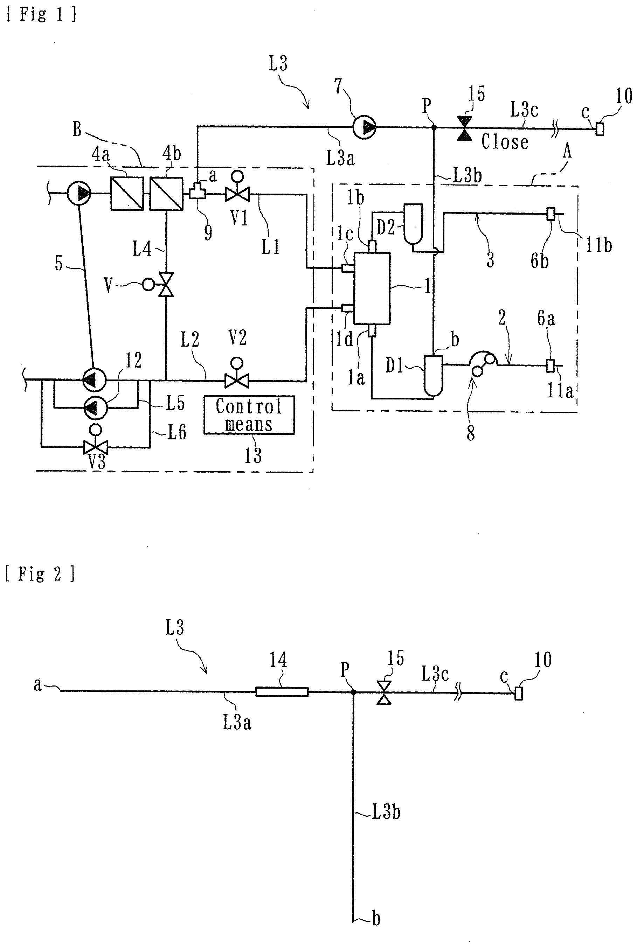

The dialysis apparatus of the present invention is applied to the hemodialysis (HD) and mainly comprises, as shown in Fig. 1, a blood circuit "A" in which an arterial blood circuit 2 and a venous blood circuit 3 are connected to a dialyzer 1 as a blood purification instrument, a dialysis apparatus main body "B" provided with a dialysate introducing line L1 and a dialysate discharging line L2, and a dialysate infusing line L3 arranged thereon a dialysate infusing pump 7.

The dialyzer 1 contains therein a blood purification membrane (hollow fiber membrane in the preferable embodiment, however including semi-permeable membrane and filtration membrane) and has a blood introducing port la for introducing blood to be dialyzed, a blood discharging port 1b for discharging dialyzed blood, a dialysate introducing port 1c for introducing the dialysate, and a dialysate discharging port 1d for discharging the used dialysate in dialysis. The dialyzer 1 can perform the dialysis for blood purification with contacting blood introduced via the blood introducing port la with the dialysate through the hollow fiber membrane.

The arterial blood circuit 2 is mainly formed of a flexible tube adapted to be connected to the blood introducing port la of the dialyzer 1 at its base end for introducing blood collected from the artery of a patient into the hollow fiber membrane within the dialyzer 1. A connector 6a for mounting an arterial puncture needle 11a is secured on the tip end of the arterial blood circuit 2 and an air trap chamber (arterial air trap chamber) D1 is arranged on the way of the arterial blood circuit 2. In addition, a blood pump 8 of peristaltic type (pump adapted to discharge blood with the flexible tube being squeezed toward a predetermined direction from its outside) is arranged on the arterial blood circuit 2 at a position nearer to the tip end than the air trap chamber D1.

Similarly to the arterial blood circuit 2, the venous blood circuit 3 is mainly formed of flexible tube and adapted to be connected to the blood discharging port 1b of the dialyzer 1 at its base end for discharging blood passed through flow routes in the hollow fiber membrane. A connector 6b for mounting a venous puncture needle 11b is secured on the tip end of the venous blood circuit 3 and an air trap chamber (venous air trap chamber) D2 is arranged on the way of the venous blood circuit 2.

The dialysate introducing line L1 and the dialysate discharging line L2 are connected respectively to the dialysate introducing port 1c and the dialysate discharging port 1d and the dialysate introduced into the dialyzer 1 from the dialysate introducing line L1 can be passed through the outside of the hollow fiber membrane and discharged through the dialysate discharging line L2. Electromagnetic valves V1 and V2 are arranged respectively on the way of the dialysate introducing line L1 and the dialysate discharging line L2. In addition, There is arranged a bypass line L4 between the dialysate introducing line L1 and the dialysate discharging line L2 for connecting them not through the dialyzer 1 and an electromagnetic valve V is arranged on the bypass line L4.

A duplex pump (supply device) 5 is arranged within the dialysis apparatus main body "B" so that it is connected to both the dialysate introducing line L1 and the dialysate discharging line L2 in order to supply prepared dialysate to the dialysate introducing line L1. Bypass lines L5, L6 bypassing the duplex pump 5 are arranged on the dialysate discharging line L2 and an ultrafiltration pump 12 for removing excessive water in blood of a patient is arranged on the bypass line L5 and an electromagnetic valve V3 is arranged on the bypass line L6. Reference numerals 4a, 4b denote filters for cleaning the dialysate. It is preferable to arrange at least one such filter, however two or more filters may be arranged as shown in the present preferable embodiments.

In performing the hemodialysis treatment, firstly the arterial puncture needle 11a and the venous puncture needle 11b are punctured into a body of a patient, then blood of a patient is extracorporeally circulated through the arterial blood circuit 2, the dialyzer 1 and the venous blood circuit 3 by driving the blood pump 8 with introducing the dialysate to the dialyzer 1 by driving the duplex pump 5, thus the blood of a patient is purified and the ultrafiltration is also performed by driving the ultrafiltration pump 12.

The T-tube 9 is arranged on the way of the dialysate introducing line L1 (between the filter 4b and the electromagnetic valve V1) and one end "a" of the dialysate infusing line L3 is connected to the T-tube 9. As shown in Fig. 2, the dialysate infusing line L3 of the present invention is a flow route formed e.g. by a flexible tube etc. and has said one end "a" to be connected to the dialysate introducing line L1 via the T-tube and the other end branched to a first branch end "b" and a second branch end "c". The first branch end "b" is connected to the air trap chamber (arterial air trap chamber) D1 and the second branch end "c" can be connected to a tip end of the arterial blood circuit 2 during the blood-returning process. A reference numeral 14 denotes a tube portion to be squeezed by the dialysate infusing pump 7, the tube portion having a large diameter and formed of a soft tube.

In the dialysate infusing line L3, it will be conveniently defined that a flow route from the one end "a" to the branch point "P" is a main flow route L3a, a flow route from the branch point "P" to the first branch end "b" is a first branch flow route L3b, and a flow rate from the branch point "P" to the second branch end "c" is a second branch flow route L3c. The tip end (i.e. the first branch end "b") of the first branch flow route L3 is previously connected to the air trap chamber D1 and a connector 10 connectable to the connector 11a of the tip end of the arterial blood circuit 2 is mounted on the tip end (i.e. second branch end "c") of the second branch flow route L3c.

The dialysate infusing pump 7 is a peristaltic pump arranged on the dialysate infusing line L3 at a connection-side of the dialysate infusing line L3 (i.e. between the one end "a" and the branch point "P" of the main flow route L3a) for supplying the dialysate of the dialysate introducing line L1 to the first branch end "b" via the first branch flow route L3b as well as to the second branch end "c" via the second branch flow route L3c. Thus it is possible to perform the dialysate infusion (pre-dialysate infusion) during the hemodialysis treatment by driving the dialysate infusing pump 7 to supply the dialysate in the dialysate introducing line L1 to the arterial blood circuit 2 via the air trap chamber (arterial air trap chamber) D1.

In addition, a valve device 15 for cutting off and opening the supply of dialysate is arranged on the dialysate infusing line L3 between the branch point P and the second branch end "c" (i.e. on the way of the second branch flow route L3c). Such a valve device 15 may be a clamp device able to cut off and open the supply of dialysate by manually clamping or unclamping the flow route or an electromagnetic valve capable of cutting off and opening the supply of the dialysate by clamping or unclamping the flow route.

In the present invention, the dialysis apparatus is structured so that the supply of dialysate is cut off during the hemodialysis treatment (including dialysate infusion) by closing the valve device 15 as shown in Fig. 1. Then, for performing the blood-return to a patient after completion of the hemodialysis treatment, the arterial puncture needle 11a is removed with keeping the venous puncture needle 11b punctured in a patient and then the connector 10 of the second branch end "c" of the dialysate infusing line L3 is connected to the connector 6a of the tip end of the arterial blood circuit 2. During the blood-returning process, flow of the dialysate is maintained by opening the valve device 15.

The blood-return is carried out as follows. Firstly, the piping of the dialysis apparatus main body "B" is separated from the blood flow route within the dialyzer 1 by opening the electromagnetic valve "V" with keeping the electromagnetic valves V1, V2 closed to form a flow route bypassing the dialyzer 1 and the electromagnetic valve V3 of the bypass line L6 is opened. Then by rotating both the blood pump 8 and the dialysate infusing pump 7 toward the normal rotational direction under this condition, the dialysate in the dialysate introducing line L1 flows into the arterial blood circuit 2 via the main flow route L3a and the second branch flow route L3c of the dialysate infusing line L3. Thus the blood-return canbe performed by replacing the dialysate with blood. According to such a structure of the dialysis apparatus of the present invention, a portion of the dialysate can flow from the branch point "P" to the first branch end "b" through the first branch flow route L3b even if the flow rate caused by driving of the blood pump 8 is higher than that caused by driving of the dialysate infusing pump 7 and thus the dialysate can be flowed into the arterial blood circuit 2 with bypassing the blood pump 8.

Also according to the present invention, the dialysis apparatus main body "B" is provided with a control device 13 comprising a microcomputer etc. electrically connected to driving control device of the blood pump 8 and the dialysate infusing pump 7. Such a control device 13 controls the drive of the blood pump 8 and the dialysate infusing pump 7 and can synchronously control them so that the rotational speed of the dialysate infusing pump 7 is higher than that of the blood pump 8 by a predetermined ratio (about 10% in the preferable embodiment) during the blood-returning process. That is, since an error is caused in actual flow rate of the dialysate infusing pump 7 and the blood pump 8 even if the driving speed of them is set, it is previously set in anticipation of the error so that the rotational speed of the dialysate infusing pump 7 is higher than that of the blood pump 8 by a predetermined ratio.

Thus, since the control device 13 can synchronously control the dialysate infusing pump 7 and the blood pump 8 so that the rotational speed of the dialysate infusing pump 7 is higher than that of the blood pump 8 by a predetermined ratio, it is possible to absorb the erroneous amount even if there would be caused an error in the flow rate of the dialysate infusing pump 7 and the blood pump 8. Accordingly it is possible to prevent back flow (flow from the first branch end "b" to the branch point "P") of blood through the flow route between the branch point "P" and the first branch end "b" of the dialysate infusing line L3 which would be caused if the real flow rate of the blood pump 8 is higher than that of the dialysate infusing pump 7. Especially according to the present invention, since the predetermined ratio is set at about 10%, it is possible to sufficiently absorb the error (about 10%) usually caused in general purpose pumps such as the blood pump 8 and the dialysate infusing pump 7.

In addition, the control device 13 of the present invention is so structured that it drives both the blood pump 8 and the dialysate infusing pump 7 at a commencement of the blood-return by the synchronous control as described above and stops the blood pump 8 after the lapse of a predetermined time and drives only the dialysate infusing pump 7. The phrase "after the lapse of a predetermined time" device a time after a duration in which the flow route between the tip end of the arterial blood circuit 2 and the air trap chamber (arterial air trap chamber) D1 has been completely replaced by dialysate and it may be a time actually obtained from an experiment or a time theoretically obtained from a volume of the flow route and a flow rate of the dialysate. This makes it possible to reduce the usage amount of dialysate during the blood-return as well as the blood-returning time as compared to the case in which the blood pump 8 and the dialysate infusing pump 7 are kept driven during the time from the start to the completion of the blood-returning process. According to the present invention, although the condition for stopping the blood pump 8 is a "time" required for completely replacing blood with dialysate, it is possible to directly detect the completion of the replacement by any concentration detecting device arranged in the blood circuit in place of measuring the "time".

According to the present invention, since the dialysate infusing line L3 is so structured that one end "a" of which is connected to the dialysate introducing line L1, the other end-side is branched out at a branch point "P" into two flow routes respectively having a first branch end "b" and a second branch end "c", the first branch end "b" is able to be connected to the arterial blood circuit 2, and the second branch end "c" is able to be connected to the tip end of the arterial blood circuit 2 during the blood-returning process, it is possible, during the blood-returning process, to allow the dialysate to be flowed through both the flow route of the dialysate infusing line L3 between the branch point "P" and the second branch end "c" and the flow route between the branch point "P" and the first branch end "b".

Accordingly, the dialysate can flow through the flow route between the branch point "P" and the first branch end "b" of the dialysate infusing line L3 even when the flow rate (discharging amount) of the blood pump 8 is lower than that of the dialysate infusing pump 7 due to the error in flow rate of the blood pump 8 and the dialysate infusing pump 7 during the blood-returning process, and thus the generation of excessive positive pressure in the flow route between the blood pump 8 and the dialysate infusing pump 7 can be prevented.

In addition, according to the present invention, since a valve device 15 for cutting off and opening the supply of dialysate is arranged on the dialysate infusing line L3 between the branch point "P" and the second branch end "c", it is possible to surely prevent the dialysate from being discharged from the second branched end "c" on the dialysate infusion time during dialysis treatment with the dialysate flow being cut off before the blood-return and opened during the blood-return by the valve device 15.

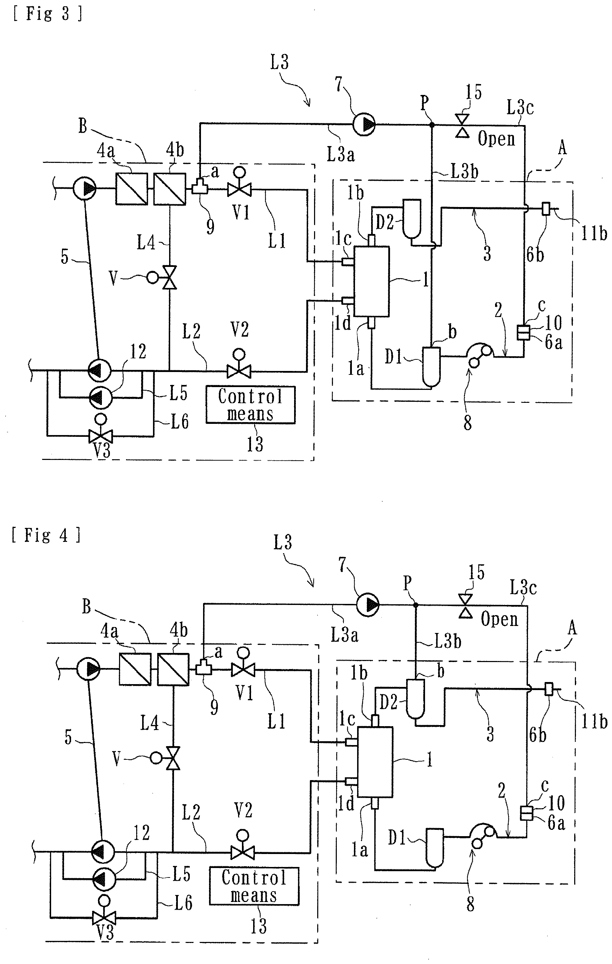

Further according to the present invention, since the first branch end "b" can be connected to an arterial air-trap chamber (arterial air trap chamber) D1 arranged on the arterial blood circuit 2, it is possible to perform the bubble removal on feeding the dialysate to the arterial blood circuit 2 during the dialysate infusion into the arterial blood circuit 2. However as shown Fig. 4, it may be possible to perform the dialysate infusion (post-dialysate infusion) by connecting the first branch end "b" to the air trap chamber (venous air trap chamber) arranged on the way of the venous blood circuit 3 and supplying the dialysate into the venous blood circuit 3 during the dialysate infusing process.

The preferable embodiments of the present invention have been described above. However, the present invention is not limited to these illustrated embodiments. For example, the dialysate infusing line may be a line of which one end "a" is connected to the dialysate discharging line L2 or a line of which first branch end "b" is connected to the venous blood circuit 3. In addition, although the first branch end "b" is connected to the air trap chamber (arterial air trap chamber) D1 or to the air trap chamber (venous air trap chamber) D2, it may be connected to a flow route itself forming the arterial blood circuit 2 (it may be connected to a flow route itself forming the venous blood circuit 3).

### Applicability in Industries

The present invention can be applied to any other dialysis apparatus to which other functions than those described in the present specification are added, if it is a dialysis apparatus comprising a dialysate infusing line, one end of which being connected to the dialysate introducing line or the dialysate discharging line, the other end-side being branched out at a branch point into two flow routes respectively having a first branch end and a second branch end, the first branch end being able to be connected to the arterial blood circuit or the venous blood circuit, and the second branch end being able to be connected to the tip end of the arterial blood circuit during the blood-returning process, and a dialysate infusing pump arranged on the dialysate infusing line at a connection-side of the dialysate infusing line relative to the dialysate introducing line or the dialysate discharging line from the branch point for supplying the dialysate of the dialysate introducing line or the dialysate discharging line to the first branch end and the second branch end.

### Explanation of Reference Numerals

- 1: dialyzer (blood purification instrument)
- 2: arterial blood circuit
- 3: venous blood circuit
- 4a, 4b: filter
- 5: duplex pump
- 6a, 6b: connector
- 7: dialysate infusing pump
- 8: blood pump
- 9: T-tube
- 10: connector
- 11a: arterial puncture needle
- 11b: venous puncture needle
- 12: ultrafiltration pump
- 13: control device
- 14: portion to be squeezed
- 15: valve device
- L1: dialysate introducing line
- L2: dialysate discharging line
- L3: dialysate infusing line
- L4: bypass line
- L5: bypass line
- L6: bypass line
- V1: electromagnetic valve
- V2: electromagnetic valve
- V3: electromagnetic valve

## Claims

1. A dialysis apparatus comprising:
a blood purification instrument (1) containing therein a blood purification membrane and formed with a blood introducing port (1a), a blood discharging port (1b), a dialysate introducing port (1c) and a dialysate discharging port (1d) for performing the dialysis purification by contacting blood with dialysate through the blood purification membrane;
an arterial blood circuit (2), the base end of which being connected to the blood introducing port (1a) of the blood purification instrument (1) and having a blood pump (8) arranged thereon;
a venous blood circuit (3), the base end of which being connected to the blood discharging port (1b) of the blood purification instrument (1);
a dialysate introducing line (L1) connected to the dialysate introducing port (1c) of the blood purification instrument (1) for introducing dialysate to the blood purification instrument (1);
a dialysate discharging line (L2) connected to the dialysate discharging port (1d) of the blood purification instrument (1) for discharging dialysate from the blood purification instrument (1); and
a dialysate supplying device for supplying prepared dialysate to the dialysate introducing line (L1) **characterized in that** the dialysis apparatus further comprises:
a dialysate infusing line (L3), one end (a) of which being connected to the dialysate introducing line (L1) or the dialysate discharging line (L2), the other end-side being branched out at a branch point (P) into two flow routes respectively having a first branch end (b) and a second branch end (c), the first branch end (b) being able to be connected to the arterial blood circuit (2) or the venous blood circuit (3), and the second branch end (c) being able to be connected to the tip end of the arterial blood circuit (2) during the blood-returning process, and
a dialysate infusing pump (7) arranged on the dialysate infusing line (L3) at a connection-side of the dialysate infusing line (L3) relative to the dialysate introducing line (L1) or the dialysate discharging line (L2) from the branch point (P) for supplying the dialysate of the dialysate introducing line (L1) or the dialysate discharging line (L2) to the first branch end (b) and the second branch end (c), wherein the dialysis apparatus further comprises a control device (13) configured to synchronously control the blood pump (8) and the dialysate infusing pump (7) so that the rotational speed of the dialysate infusing pump (7) is higher than that of the blood pump (8) by a predetermined ratio during the blood-returning process into the arterial blood circuit (2).

2. A dialysis apparatus of claim 1 wherein a valve device (15) for cutting off or opening the supply of dialysate is arranged on the dialysate infusing line (L3) between the branch point (P) and the second branch end (c).

3. A dialysis apparatus of claim 1 or 2 wherein the first branch end (b) can be connected to an arterial air-trap chamber (D1) arranged on the arterial blood circuit (2) or a venous air-trap chamber (D2) arranged on the venous blood circuit (3).

4. A dialysis apparatus of claim 1 wherein the predetermined ratio is about 10%

5. A dialysis apparatus of claim 1 or 4 wherein the control device controls the blood pump (8) and the dialysate infusing pump (7) so that both the blood pump (8) and the dialysate infusing pump (7) are driven at the start of the blood-return and only the dialysate infusing pump (7) is driven with stopping the blood pump (8) after the lapse of a predetermined time.

## Patentansprüche

1. Dialysegerät, aufweisend:
ein Blutreinigungsinstrument (1), das eine Blutreinigungsmembran enthält und mit einer Bluteintragsöffnung (1a), einer Blutaustragsöffnung (1b), einer Dialysat-Eintragsöffnung (1c) und einer Dialysat-Austragsöffnung (1d) ausgebildet ist, um die Dialysereinigung durchzuführen, und zwar dadurch, dass Blut mit Dialysat über die Blutreinigungsmembran in Kontakt gebracht wird;
einen arteriellen Blutkreislauf (2), dessen Basisende an die Bluteintragsöffnung (1a) des Blutreinigungsinstruments (1) angeschlossen ist und eine daran angeordnete Blutpumpe (8) aufweist;
einen venösen Blutkreislauf (3), dessen Basisende an die Blutaustragsöffnung (1b) des Blutreinigungsinstruments (1) angeschlossen ist;
eine Dialysat-Eintragsleitung (L1), die an die Dialysat-Eintragsöffnung (1c) des Blutreinigungsinstruments (1) angeschlossen ist, und zwar für ein Einbringen von Dialysat in das Blutreinigungsinstrument (1);
eine Dialysat-Austragsleitung (L2), die an die Dialysat-Austragsöffnung (1d) des Blutreinigungsinstruments (1) angeschlossen ist, und zwar für ein Abführen von Dialysat aus dem Blutreinigungsinstrument (1); und
eine Dialysat-Zuführvorrichtung für ein Zuführen von vorbereitetem Dialysat zur Dialysat-Eintragsleitung (L1), **dadurch gekennzeichnet, dass** das Dialysegerät weiter aufweist:
eine Dialysat-Infundierleitung (L3), deren eines Ende (a) an die Dialysat-Eintragsleitung (L1) oder die Dialysat-Austragsleitung (L2) angeschlossen ist, wobei sie aufseiten des anderen Endes an einem Verzweigungspunkt (P) in zwei Strömungswege verzweigt ist, die ein erstes Verzweigungsende (b) bzw. ein zweites Verzweigungsende (c) aufweisen, wobei das erste Verzweigungsende (b) fähig ist, an den arteriellen Blutkreislauf (2) oder den venösen Blutkreislauf (3) angeschlossen zu werden, und das zweite Verzweigungsende (c) fähig ist, während des Blutrückführprozesses an das äußere Ende des arteriellen Blutkreislaufs (2) angeschlossen zu werden, und
eine Dialysat-Infundierpumpe (7), die auf der Dialysat-Infundierleitung (L3) bei einer Verbindungsseite der Dialysat-Infundierleitung (L3) bezüglich der Dialysat-Eintragsleitung (L1) oder der Dialysat-Austragsleitung (L2) von dem Verzweigungspunkt (P) angeordnet ist, um das Dialysat der Dialysat-Eintragsleitung (L1) oder der Dialysat-Austragsleitung (L2) dem ersten Verzweigungsende (b) und dem zweiten Verzweigungsende (c) zuzuführen, wobei das Dialysegerät weiter eine Steuervorrichtung (13) aufweist, die konfiguriert ist, die Blutpumpe (8) und die Dialysat-Infundierpumpe (7) so zu steuern, dass die Drehzahl der Dialysat-Infundierpumpe (7) um einen vorbestimmten Quotienten größer als die der Blutpumpe (8) ist, und zwar während des Blutrückführprozesses in den arteriellen Blutkreislauf (2).

2. Dialysegerät nach Anspruch 1, wobei eine Ventilvorrichtung (15) zum Absperren oder Öffnen der Zufuhr von Dialysat auf der Dialysat-Infundierleitung (L3) zwischen dem Verzweigungspunkt (P) und dem zweiten Verzweigungsende (c) angeordnet ist.

3. Dialysegerät nach Anspruch 1 oder 2, wobei das erste Verzweigungsende (b) an eine arterielle Luftfangkammer (D1), die in dem arteriellen Blutkreislauf (2) angeordnet ist, oder eine venöse Luftfangkammer (D2) angeschlossen sein kann, die in dem venösen Blutkreislauf (3) angeordnet ist.

4. Dialysegerät nach Anspruch 1, wobei der vorbestimmte Quotient ca. 10 % beträgt.

5. Dialysegerät nach Anspruch 1 oder 4, wobei die Steuervorrichtung die Blutpumpe (8) und die Dialysat-Infundierpumpe (7) so steuert, dass sowohl die Blutpumpe (8) als auch die Dialysat-Infundierpumpe (7) zu Beginn der Blutrückführung angetrieben werden, und nach Verstreichen einer vorbestimmten Zeit lediglich die Dialysat-Infundierpumpe (7) angetrieben wird und dabei die Blutpumpe (8) gestoppt ist.

## Revendications

1. Appareil de dialyse comprenant :
un instrument de purification de sang (1) contenant à l'intérieur de ce dernier, une membrane de purification de sang et formé avec un orifice d'introduction de sang (1a), un orifice de décharge de sang (1b), un orifice d'introduction de dialysat (1c) et un orifice de décharge de dialysat (1d) pour réaliser la purification par dialyse en mettant en contact le sang avec le dialysat par le biais de la membrane de purification de sang ;
un circuit de sang artériel (2), dont l'extrémité de base est raccordée à l'orifice d'introduction de sang (1a) de l'instrument de purification de sang (1) et ayant une pompe sanguine (8) agencée sur ce dernier ;
un circuit de sang veineux (3), dont l'extrémité de base est raccordée à l'orifice de décharge de sang (1b) de l'instrument de purification de sang (1) ;
une ligne d'introduction de dialysat (L1) raccordée à l'orifice d'introduction de dialysat (1c) de l'instrument de purification de sang (1) pour introduire le dialysat dans l'instrument de purification de sang (1) ;
une ligne de décharge de dialysat (L2) raccordée à l'orifice de décharge de dialysat (1d) de l'instrument de purification de sang (1) pour décharger le dialysat de l'instrument de purification de sang (1);et
un dispositif d'alimentation en dialysat pour fournir le dialysat préparé à la ligne d'introduction de dialysat (L1),
**caractérisé en ce que** l'appareil de dialyse comprend en outre :
une ligne de perfusion de dialysat (L3), dont une extrémité (a) est raccordée à la ligne d'introduction de dialysat (L1) ou à la ligne de décharge de dialysat (L2), l'autre extrémité étant ramifiée au niveau d'un point de ramification (P) en deux voies d'écoulement ayant respectivement une première extrémité de ramification (b) et une seconde extrémité de ramification (c), la première extrémité de ramification (b) pouvant être raccordée au circuit de sang artériel (2) ou au circuit de sang veineux (3), et la seconde extrémité de ramification (c) pouvant être raccordée à l'extrémité du circuit de sang artériel (2) pendant le processus de retour sanguin, et
une pompe de perfusion de dialysat (7) agencée sur la ligne de perfusion de dialysat (L3) d'un côté de raccordement de la ligne de perfusion de dialysat (L3) par rapport à la ligne d'introduction de dialysat (L1) ou la ligne de décharge de dialysat (L2) à partir du point de ramification (P) pour amener le dialysat de la ligne d'introduction de dialysat (L1) ou de la ligne de décharge de dialysat (L2) à la première extrémité de ramification (b) et à la seconde extrémité de ramification (c), l'appareil de dialyse comprenant en outre un dispositif de commande (13) configuré pour commander de manière synchrone la pompe sanguine (8) et la pompe de perfusion de dialysat (7) de sorte que la vitesse de rotation de la pompe de perfusion de dialysat (7) est supérieure à celle de la pompe sanguine (8) par un rapport prédéterminé pendant le processus de retour de sang dans le circuit de sang artériel (2).

2. Appareil de dialyse selon la revendication 1, dans lequel un dispositif de vanne (15) pour fermer ou ouvrir l'alimentation de dialysat est agencé sur la ligne de perfusion de dialysat (L3) entre le point de ramification (P) et la seconde extrémité de ramification (c).

3. Appareil de dialyse selon la revendication 1 ou 2, dans lequel la première extrémité de ramification (b) peut être raccordée à une chambre de piège à air artériel (D1) agencée sur le circuit de sang artériel (2) ou une chambre de piège à air veineux (D2) agencée sur le circuit de sang veineux (3).

4. Appareil de dialyse selon la revendication 1, dans lequel le rapport prédéterminé est d'environ 10 %.

5. Appareil de dialyse selon la revendication 1 ou 4, dans lequel le dispositif de commande commande la pompe sanguine (8) et la pompe de perfusion de dialysat (7) de sorte qu'à la fois la pompe sanguine (8) et la pompe de perfusion de dialysat (7) sont entraînées au début du retour sanguin et seule la pompe de perfusion de dialysat (7) est entraînée avec l'arrêt de la pompe sanguine (8) après l'écoulement d'un laps de temps prédéterminé.
